# EUROPÄISCHE PATENTSCHRIFT

(11) **EP 0 406 606 B1**
(45) Veröffentlichungstag und Bekanntmachung des Hinweises auf die Patenterteilung: **01.09.1993**
(21) Anmeldenummer: 90111399.3
(22) Anmeldetag: 16.06.1990
(51) Int. Cl.: A61F 2/38

(54) **Kniegelenk-Endoprothese**
Knee joint endoprosthesis
Endoprothèse de l'articulation du genou

(30) Priorität: 07.07.1989 DE 3922294
(43) Veröffentlichungstag der Anmeldung: 09.01.1991
(73) Patentinhaber: ESKA MEDICAL LÜBECK MEDIZINTECHNIK GmbH & Co., D-23556 Lübeck (DE)
(72) Erfinder: Moser, Heinz, D-6227 Oestrich-Winkel (DE)
(74) Vertreter: Weiss, Christian, Dipl.-Ing.

(56) Entgegenhaltungen:
- DE-B- 2 452 412
- GB-A- 1 333 412
- US-A- 4 215 439
- US-A- 4 224 697

## Beschreibung

Die Erfindung betrifft eine Kniegelenk-Endoprothese gemäß dem Oberbegriff des Anspruches 1, wie sie beispielsweise aus der DE-B-24 52 412 bekannt ist.

Eine derartige Endoprothese ist ferner bekannt unter der Bezeichnung "Insall/Burstein" Total Knee System aus einem Prospekt der Firma Zimmer, USA.

Als Führungselement für das Femurteil dient bei dieser gattungsgemäßen Endoprothese ein zwischen den Gleitflächen des Tibiateiles vorgesehener einfacher Steg, welcher in gestreckter Lage des Gelenkes durch eine Öffnung in den zwischen den Gleitkufen des Femurteiles begrenzten Hohlraum eingreift. Mit zunehmender Beugung des Gelenkes kommt dieser Steg außer Eingriff mit dieser Öffnung. Eine Führung der Gleitkufen erfolgt ausschließlich durch eine Anlage der planen Seitenflächen des besagten Steges an den ebenfalls planen Führungsfläche des Femurteiles.

Als nachteilig wird bei dieser Endoprothese die nicht befriedigende Führungsfunktion sowie eine nur bedingt vorhandene Ablastfunktion des Steges bei Lastwechsel-Bewegungen empfunden. Hohe Abriebserscheinungen an den sich gegeneinander abstützenden Teilen sowie eine physiologisch nur unzureichend nachempfundene Bewegung des Gelenkes sind die Folge des Aufbaues der bekannten Endoprothese.

Hier Abhilfe zu schaffen ist die Aufgabe der vorliegenden Erfindung. Darüber hinaus ist ein weiterer Aspekt der Erfindung darin zu sehen, die Handhabung bei der Implantation oder gegebenenfalls beim Austausch verschlissener Teile zu erleichtern.

Gelöst die Aufgabe durch die Kniegelenk-Endoprothese mit den Merkmalen des Anspruches 1. Weitere vorteilhafte Ausgestaltungen ergeben sich aus den Unteransprüchen.

Erfindungsgemäß ist das Tibiateil mit einem mit seiner Hauptachse femurwärts ausgerichteten Doppelkegelstumpf als Führungs- und Ablastelement versehen. In dem zwischen den Gleitkufen des Femurteiles begrenzten Hohlraum sind den Neigungswinkeln beider den Doppelkegelstumpf bildenden Halbkegelstümpfe entsprechend geneigte und diese teilweise umfassende Führungsflächenpaare ausgebildet, an denen die Kegelflächen der Halbkegelstümpfe in gleitende Anlage kommen. In Abhängigkeit von der Wahl der Größe der Spalte zwischen dem Doppelkegelstumpf und den Führungsflächen im Hohlraum findet eine Ablastung bei Ausführung von Lastwechsel-Bewegungen, also von seitlich auf das Gelenk einwirkende Belastungen, oder aber zusätzlich eine die Gleitkufen und Gleitflächen des Femur- bzw. Tibiateiles ent lastende Teilaufnahme auf das Gelenk entlang dessen Hauptachse einwirkender Kräfte statt. Letzteres wird insbesondere der Fall sein bei nur sehr kleinen Spaltöffnungen zwischen dem Doppelkegelstumpf und den Führungsflächenpaaren. Hierdurch ergibt sich eine Reduzierung der Abriebserscheinungen an den Gleitkufen und Gleitflächen. In jedem Falle aber sind die Spaltöffnungen so gewählt, daß Lastwechsel-Bewegungen von dem Doppelkegelstumpf abgelastet werden.

Der physiologische Bewegungsablauf eines gesunden Kniegelenkes kann mit der vorliegenden Endoprothese besonders gut nachempfunden werden, wenn die beiden Gleitflächen des Tibiateiles von zwei den Doppelkegelstumpf einfassenden,von dorsal nach ventral ausgerichtet verlaufenden Stegen getrennt sind, wobei der dorsal gelegene - also in Laufrichtung von hinten gesehene - Steg eine größere Breite als der ventral gelegene Steg aufweist. Hierdurch wird bei Beugeposition des Gelenkes ein Spiel zwischen den Gelenkteilen ermöglicht, wie es sich auch bei einem gesunden Knie findet. Im gestreckten Zustand des Gelenkes hingegen ist dieses - wie ein natürliches Knie - nahezu spielfrei. Zwischen diesen beiden Extrempositionen vergrößert sich das Spiel ausgehend von der Strecklage stetig.

Die Neigungswinkel beider den Doppelkegelstumpf bildenden Halbkegelstümpfe sind nicht immer notwendigerweise gleich. Für eine symmetrische Flächenpressung an dem Doppelkegelstumpf ist es aber vorteilhaft, wenn beide Neigungswinkel gleich groß sind. In diesem Falle sind die Führungsflächenpaare im Hohlraum des Femurteiles ebenfalls entsprechend den Neigungswinkeln der Halbkegelstümpfe in gleichem Maße geneigt.

In einfacher Weise kann der Doppelkegelstumpf mit einer ihn axial durchgreifenden Schraube mit dem Tibiateil lösbar verbunden sein. Dies gestattet im Eventualfall eines nicht mehr zu vertretenden Abriebs nach längerem Einsatz den Ersatz des Doppelkegelstumpfes, ohne das ganze in der Tibia verankerte Tibiateil auswechseln zu müssen.

Der Doppelkegelstumpf besteht vorzugsweise aus einem abriebfesten Kunststoff wie Polyäthylen oder Polyazetal. Zur Verstärkung des Doppelkegelstumpfes kann dieser mit einer Armierung aus Metall versehen sein.

Vorteilhaft ist es, die Gleitflächen des Tibiateiles in einer lösbar mit diesem verbundenen Kunststoffauflage mit niedrigem Reibungskoeffizient, etwa aus Poyäthylen, auszubilden. Das übrige Tibiateil besteht in diesem Falle ansonsten aus Metall. Diese Ausführungsform gestattet einen nach längerem Einsatz des Gelenkes durch etwaig auftretenden Abrieb erforderlichen Ersatz der Kunststoffauflage, ohne das Tibiateil im übrigen ersetzen zu müssen.

Vorteilhaft ist es weiterhin, das Femurteil zweistückig auszubilden. Ein erstes Teil weist hierbei einen Stiel auf, mittels dessen es im Femurknochen verankerbar ist. Es weist Anlageflächen für ein femurwärts komplementär ausgebildetes zweites Teil auf, welches mittels einer konischen Verklemmung mit dem ersten Teil kraftschlüssig verbindbar ist. Im übrigen wird zwischen beiden Teilen ein Formschluß hergestellt. Das zweite Teil trägt in diesem Falle die Gleitkufen.

Die Zweistückigkeit des Femurteiles erleichtert zum einen die Implantation der Endoprothese. Erst nach der Implantation des ersten Teiles im Femurknochen wird das zweite Teil mit dem ersten Teil verbunden und mit dem Tibiateil in Eingriff gebracht. Zum anderen aber wird der Ersatz des Gleitkufenteiles ermöglicht, ohne daß im Femurknochen verankerte Teil entfernen zu müssen.

Das Femurteil wird in allen Ausführungsformen im übrigen vorzugsweise aus Metall bestehen, um den auftretenden Belastungen standhalten zu können.

Im übrigen kann das Femurteil so ausgebildet sein, daß es Anschlagsflächen aufweist, an denen die Teile des resizierten Knochens zur Anlage kommen. Hierdurch ist eine stabile und definierte Einbaulage möglich. Wenn diese Flächen mit einer offenzelligen metallischen Struktur versehen sind, können Knochenbälkchen in diese einwachsen, um dem Teil einen sicheren Langzeithalt zu verleihen.

Auch alle übrigen mit Knochenmaterial unmittelbar in Berührung kommenden Teile der Endoprothese, also insbesondere auch der Stiel des Femurteiles sowie des Tibiateiles können vorteilhafterweise mit einer offenzelligen metallischen Struktur versehen sein, um ein Einwachsen von Knochenmaterial in diese zu ermöglichen.

Die Erfindung wird anhand eines Ausführungsbeispieles gemäß der Zeichnungen näher erläutert. Hierbei zeigt:
- Figur 1: eine perspektivische Ansicht der Teile der Kniegelenk-Endoprothese in einer Ausführungsform mit zweistückigem Femurteil,
- Figur 2: eine perspektivische Schnittansicht der Teile aus Figur 1,
- Figur 3: eine perspektivische Ansicht (a) des in dem Femurknochen zu verankernden Teiles der Endoprothese, dessen Ansicht (b) und in Aufsicht (c),
- Figur 4: eine perspektivische Ansicht (a) des die Gleitkufen tragenden Teiles des Femurteiles der Endoprothese, dessen Schnittansicht (b) und in Aufsicht (c),
- Figur 5: eine perspektivische Ansicht (a) des Tibiateiles ohne Doppelkegelstumpf, dessen Ansicht (b) und in Aufsicht (c),und
- Figur 6: eine Schnittansicht der zusammengefügten Endoprothese in der Ebene des Doppelkegelstumpfes.

Nachfolgend sind gleiche Teile mit denselben Bezugsziffern bezeichnet.

In den Figuren ist ausschließlich eine Ausführungsform der Kniegelenk-Endoprothese dargestellt, bei der das Femurteil zweistückig ausgebildet ist. Hierauf beschränkt sich die Erfindung jedoch nicht.

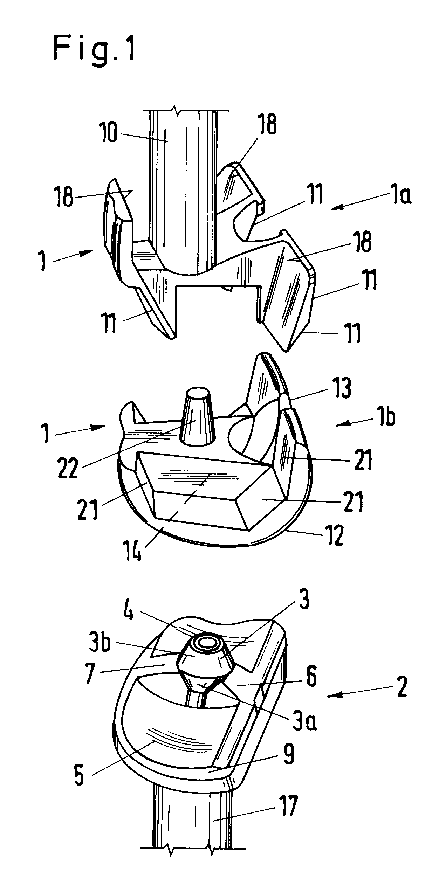

In Figur 1 ist eine perspektivische Ansicht der Kniegelenk-Endoprothese dargestellt, bestehend aus dem hier zweistückigen Femurteil 1 mit den Teilen 1a und 1b, sowie aus dem Tibiateil 2.

Das Teil 1a weist einen Stiel 10 auf, der in dem Femurknochen verankerbar ist. Es ist mit Anschlagsflächen 18 versehen, an denen Teile des für die Implantation vorbereiteten Femurknochens zur Anlage kommen. Tibiawärts weist das Teil 1a Anlageflächen 11 auf, welche nach Zusammenfügung des Teiles 1a mit dem Teil 1b auf entsprechende Anlageflächen 21 formschlüssig zum Liegen kommen.

Mittels eines Klemmkonus 22 am Teil 1b und einer entsprechenden Klemmhülse 26 sind beide Teile 1a und 1b kraftschlüssig verbindbar, um das Femurteil 1 zu bilden.

Das Teil 1b trägt die Gleitkufen 12, 13. Zwischen beiden Gleitkufen 12,13 verläuft ein tibiawärts hohler Steg, durch den ein Hohlraum 14 begrenzt ist. Wie weiter unten dargelegt wird, sind in diesem Hohlraum 14 Führungsflächenpaare ausgebildet.

Das Tibiateil weist ebenfalls einen im Knochen verankerbaren Stiel 17 auf. Es weist zwei Gleitflächen 4,5 auf, auf denen die Gleitkufen 12,13 des Femurteiles 1 abrollen und abgleiten können. Im gezeigten Ausführungsbeispiel sind die Gleitflächen 4,5 in einer Kunststoffauflage 9 ausgebildet.

Als Führungs- und Ablastelement dient der Doppelkegelstumpf 3, mit dem das Tibiateil 2 versehen ist. Dieser besteht aus zwei an ihrer Basis miteinander verbundenen Halbkegelstümpfen 3a, 3b.

Beide Gleitflächen 4,5 sind von dorsal nach ventral ausgerichtet verlaufenden Stegen 6,7 getrennt, welche den Doppelkegelstumpf teilweise einfassen. Dabei ist der dorsal gelegene Steg 6 breiter ausgebildet als der ventral gelegene Steg 7. Hierdurch wird ein Spiel des Gelenkes in der Beugeposition erzielt, wohingegen das Gelenk in der Streckposition nahezu spielfrei ist.

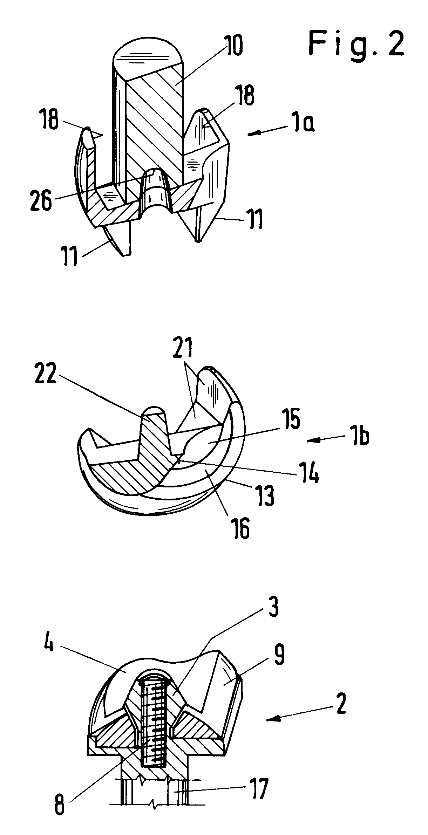

In Figur 2 sind perspektivische Schnittansichten der Teile der Endoprothese aus Figur 1 dargestellt.

Deutlich erkennbar ist im Schnittbild des Teiles 1a die konische Klemmhülse 26, in welche der Klemmkonus 22 des Teiles 1b zur Herstellung einer konischen Klemmverbindung führbar ist.

Im Schnittbild des Teiles 1b sind die Führungsflächen 15,16 im Hohlraum 14 angedeutet, die jeweils in diesen hineingeneigt sind, und zwar unter Winkeln, die den Neigungswinkeln beider Halbkegelstümpfe 3a, 3b entsprechen, so daß die Kegelflächen des Halbkegelstumpfes 3a an der Führungsfläche 16 und jene des Halbkegelstumpfes 3b an der Führungsfläche 15 in gleitende Anlage kommen können.

Der Doppelkegelstumpf 3 ist im gezeigten Ausführungsbeispiel mit einer ihn axial durchgreifenden Schraube 8 mit dem Tibiateil 2 lösbar verbunden. Die Schraube 8 sichert darüber hinaus den Sitz der Kunststoffauflage 9 auf dem Tibiateil 2, welches im übrigen aus Metall bestehen kann.

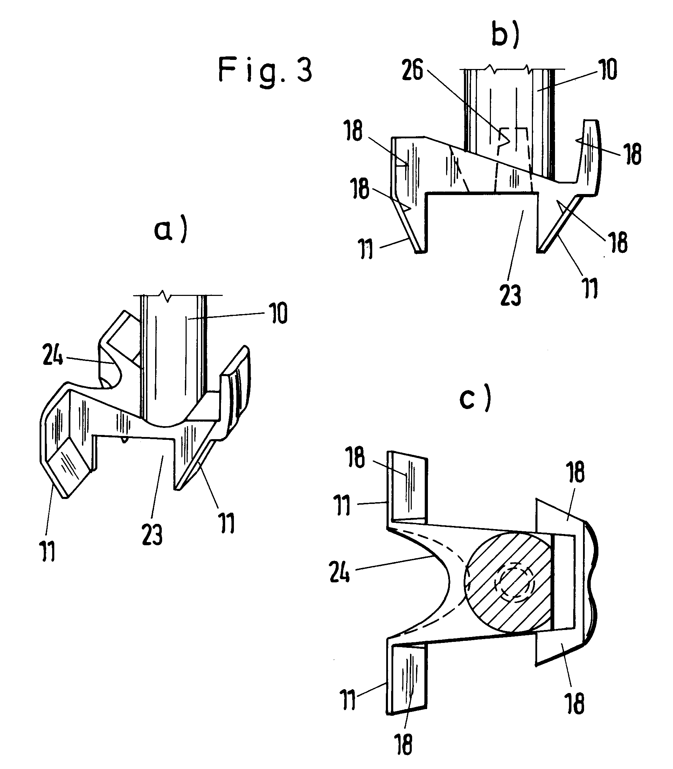

Aus Figur 3 sind weitere Einzelheiten des Teiles 1a ersichtlich.

Die Ausnehmung 23, die von den die Anschlagsflächen 18 sowie die Anlageflächen 11 tragenden Laschen begrenzt ist, ist so ausgebildet, daß sie beim Zusammenbau über den den Hohlraum 14 im Teil 1a begrenzenden Steg formschlüssig greifen kann.

Dorsal weist das Teil 1a eine Aussparung 24 von einer solchen Form auf, daß es über den Doppelkegelstumpf 3 führbar ist, und zwar nur in extremer Beugelage des Gelenkes während der Implantation.

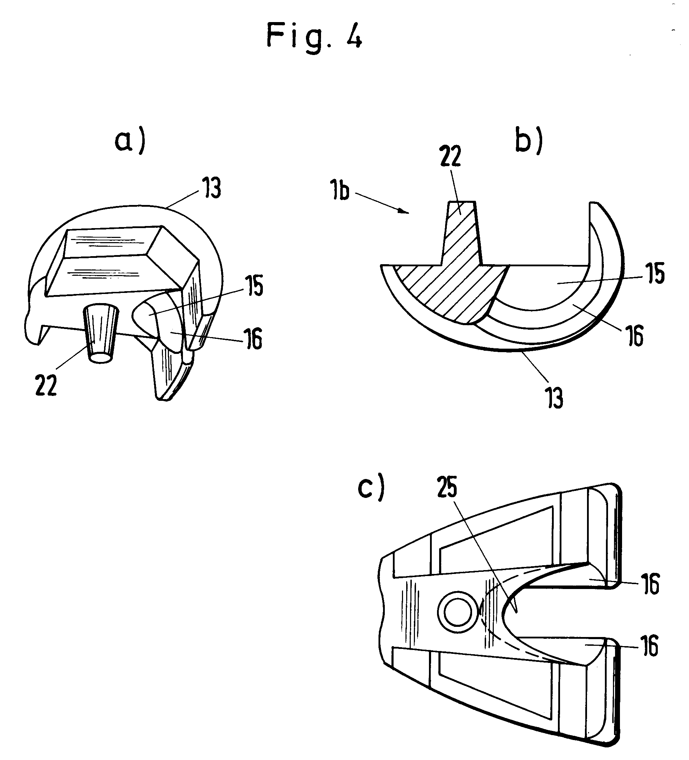

Aus Figur 4 sind weitere Einzelheiten des Teiles 1b ersichtlich. Wie oben schon erwähnt, sind die Führungsflächenpaare 15,16 im Hohlraum 14 vorgesehen, die den Neigungswinkeln der Halbkegelstümpfe 3a, 3b entsprechend in das Hohlrauminnere geneigt sind. Eine dorsal vorgesehene Aussparung 25 ist entsprechend der Aussparung 24 im Teil 1a dimensioniert und gestattet erst das Hinüberführen des Femurteiles 1 über den Doppelkegelstumpf 3.

Die Gleitkufen 12, 13 des Teiles 1b sind in bekannter Weise bogenförmig gestaltet.

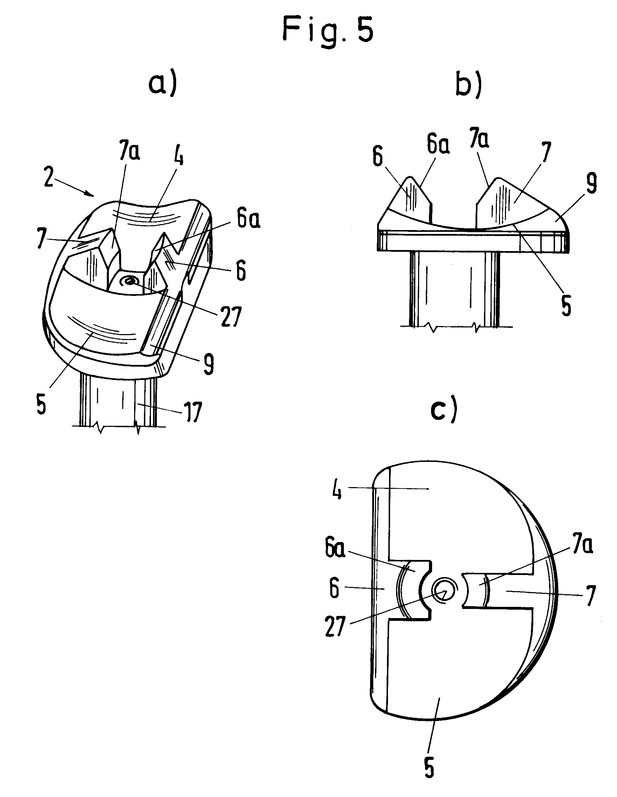

Aus Figur 5 sind weitere Einzelheiten des Tibiateiles 2 veranschaulicht. Aus Darstellungsgründen ist hier der Doppelkegelstumpf weggelassen.

Deutlich erkennbar ist die Gewindebohrung 27, in welche eine Schraube 8 zur lösbaren Befestigung des Doppelkegelstumpfes und zur Fixierung der Kunststoffauflage 9 auf dem Tibiateil 2 schraubbar ist. Die Gleitflächen 4,5 sind in der Kunststoffauflage 9 in bekannter Weise bogenförmig ausgebildet.

Die die Gleitflächen 4,5 trennenden Stege 6,7 sind an ihren Stirnflächen 6a, 7a abgeschrägt, und zwar in einem Winkel, welcher dem Neigungswinkel des Halbkegelstumpfes 3a weitgehend entspricht. Hierdurch wird eine Einfassung des Doppelkegelstumpfes 3 erreicht.

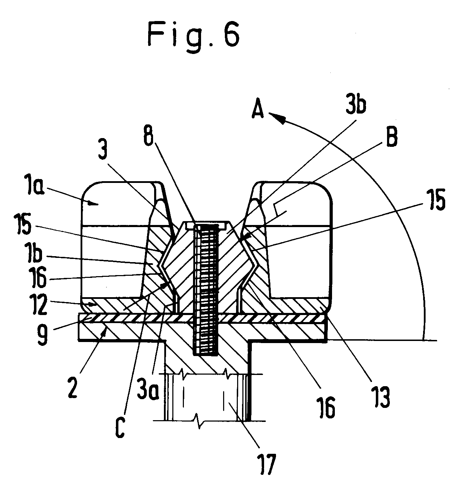

Figur 6 zeigt die zusammengefügte Endoprothese im Schnitt. Deutlich erkennbar ist, daß der Doppelkegelstumpf 3 von den Führungsflächenpaaren 15, 16 regelrecht gefangen ist. Ein Ausbrechen der Femurteile 1a, 1b unter Belastung ist demnach nicht möglich. Gleichwohl ist aber eine Verschwenkbewegung des Gelenkes gewährleistet.

Der Vorteil der Ausbildung des Führungs- und Ablastelementes als Doppelkegelstumpf 3 wird besonders deutlich, wenn das Gelenk unter einer Lastwechsel-Bewegung steht, wie dies mit dem Pfeil A angedeutet ist. In diesem Falle werden die dabei auftretenden Kräfte auf den Doppelkegelstumpf 3 über die eine Führungsfläche des Führungsflächenpaares 15 und die Kegelfläche des oberen Halbkegelstumpfes 3b in Richtung des Pfeiles B sowie über die eine Führungsfläche des Führungsflächenpaares 16 und die Kegelfläche des unteren Halbkegelstumfpes 3a in Richtung des Pfeiles C übertragen. Es ist verständlich, daß die Kräfte in Richtung der Pfeile B und C betragsmäßig gleich groß sind, jedoch entgegengerichtet wirken. Die Kräfte heben sich demnach auf, so daß die übrigen Teile des Gelenkes, ja nicht einmal die Schraube 8,einer Belastung ausgesetzt sind.

Im gezeigten Ausführungsbeispiel sind die Neigungswinkel beider Halbkegelstümpfe in etwa gleich groß gewählt.

Hierdurch wird bei einer Lastwechsel-Bewegung eine gleiche Flächenpressung auf die Flächen der Halbkegelstümpfe 3a, 3b erreicht.

Wenn die Spaltöffnung zwischen dem Führungsflächenpaar 15 und dem oberen Halbkegelstumpf 3b beispielsweise klein genug gewählt wird, so kann eine zusätzliche, die Gleitkufen 12, 13 und Gleitflächen 4,5 entlastende Teilaufnahme auf das Gelenk entlang dessen Hauptachse einwirkender Kräfte stattfinden.

## Patentansprüche

1. Kniegelenk-Endoprothese, bestehend aus einem durch einen Stiel (10) zu verankernden Femurteil (1), welches mit zwei Gleitkufen (12,13) versehen ist, die zwischen sich einen von ventral nach dorsal verlaufenden tibiawärts offenen Hohlraum (14) begrenzen, und aus einem ebenfalls durch einen Stiel (17) zu verankernden Tibiateil (2), welches zwei Gleitflächen (4,5) aufweist, auf denen die Gleitkufen (12,13) des Femurteiles (1) eine Abroll- und Gleitbewegung ausführen können,
dadurch gekennzeichnet,
daß das Tibiateil (2) mit einem mit seiner Hauptachse femurwärts ausgerichteten Doppelkegelstumpf (3) als Führungs- und Ablastelement versehen ist, und
daß im Hohlraum (14) des Femurteiles (1) den Neigungswinkeln beider den Doppelkegelstumpf (3) bildenden Halbkegelstümpfe (3a,3b) entsprechend geneigte und diese teilweise umfassende Führungsflächenpaare (15,16) ausgebildet sind, an denen die Kegelflächen der Halbkegelstümpfe (3a,3b) in gleitende Anlage kommen.

2. Kniegelenk-Endoprothese nach Anspruch 1, dadurch gekennzeichnet, daß die beiden Gleitflächen (4,5) des Tibiateiles (2) von zwei den Doppelkegelstumpf (3) einfassenden, von dorsal nach ventral ausgerichtet verlaufenden Stegen (6,7) getrennt sind, wobei der dorsal gelegene Steg (6) eine größere Breite als der ventral gelegene Steg (7) aufweist.

3. Kniegelenk-Endoprothese nach Anspruch 1 oder 2, dadurch gekennzeichnet, daß die Neigungswinkel der beiden Halbkegelstümpfe (3a,3b) im wesentlichen gleich sind.

4. Kniegelenk-Endoprothese nach einem der Ansprüche 1 bis 3, dadurch gekennzeichnet, daß der Doppelkegelstumpf (3) mit einer ihn axial durchgreifenden Schraube (8) mit dem Tibiateil (2) lösbar verbunden ist.

5. Kniegelenk-Endoprothese nach einem der Ansprüche 1 bis 4, dadurch gekennzeichnet, daß der Doppelkegelstumpf (3) aus einem abriebfesten Kunststoff besteht.

6. Kniegelenk-Endoprothese nach einem der Ansprüche 1 bis 5, dadurch gekennzeichnet, daß die Gleitflächen (4,5) des Tibiateiles (2) in einer lösbar mit diesem verbundenen Kunststoffauflage (9) mit niedrigem Reibungskoeffizienten ausgebildet sind.

7. Kniegelenk-Endoprothese nach einem der Ansprüche 1 bis 6, dadurch gekennzeichnet, daß das Femurteil (1) aus zwei Teilen (1a,1b) besteht, wobei das erste Teil (1a) mit einem Stiel (10) verankerbar ist und Anlageflächen (11) für das zweite Teil (1b) aufweist, welches mit dem ersten Teil (1a) mittels einer konischen Verklemmung verbindbar ist und die Gleitkufen (12,13) trägt.

## Claims

1. Knee joint endoprosthesis comprising a femur part (1) to be anchored by means of a column (10) and which is provided with two runners (12, 13) which between them limit an open cavity (14) extending ventrally to dorsally in the direction of the tibia, and a tibia part (2) likewise to be anchored by means of a column (17) and which has two sliding surfaces (4, 5) on which the runners (12, 13) of the femur part (1) can execute a rolling and sliding motion, characterised in that the tibia part (2) is provided with a double frustum (3), the main axis of which is aligned in the direction of the femur, as a guide and unloading element, and in that guide surface pairs (15, 16) having an appropriate angle to the slopes of both semi-frustums (3a, 3b) forming the double frustum (3) and partly surrounding them, are constructed in the cavity (14) of the femur part (1), on which guide surface pairs (15, 16) the conical surfaces of the semi-frustums (3a, 3b) come into sliding contact.

2. Knee joint endoprosthesis according to claim 1, characterised in that the two sliding surfaces (4, 5) of the tibia part (2) are separated by two crosspieces (6, 7) extending to be aligned dorsally to ventrally and surrounding the double frustum (3), wherein the dorsally laid crosspiece (6) has a greater width than the ventrally laid crosspiece (7).

3. Knee joint endoprosthesis according to claim 1 or 2, characterised in that the slope of the two semi-frustums (3a, 3b) are essentially equal.

4. Knee joint endoprosthesis according to one of claims 1 to 3, characterised in that the double frustum (3) is releasably connected to the tibia part (2) by means of a screw (8) penetrating it axially.

5. Knee joint endoprosthesis according to one of claims 1 to 4, characterised in that the double frustum (3) consists of a non-abrasive plastic.

6. Knee joint endoprosthesis according to one of claims 1 to 5, characterised in that the sliding surfaces (4, 5) of the tibia part (2) are constructed in a plastic support (9) releasably connected to the tibia part (2) and having low friction coefficients.

7. Knee joint endoprosthesis according to one of claims 1 to 6, characterised in that the femur part (1) comprises two parts (1a, 1b), wherein the first part (1a) can be anchored by means of a column (10) and has support surfaces (11) for the second part (1b) which can be connected to the first part (1a) by means of a conical lock and supports the runners (12, 13).

## Revendications

1. Endoprothèse de l'articulation du genou comportant un implant fémoral (1) pouvant être ancré grâce à une tige (10), pourvu de deux patins de glissement (12, 13) qui délimitent entre eux une cavité (14) ouverte s'étendant du côté ventral vers le côté dorsal en direction du tibia, et comportant un implant tibial (2) pouvant également être ancré grâce à une tige (17), présentant deux surfaces de glissement (4, 5), sur lesquels les patins de glissement (12, 13) de l'implant fémoral (1) peuvent effectuer un mouvement de roulement et de glissement, caractérisée en ce que l'implant tibial (2) est pourvu d'un double tronc de cône (3) dont l'axe principal est orienté en direction du fémur, servant d'élément de guidage et de soutien, et en ce que dans la cavité (14) de l'implant fémoral (1), sont formées des paires de surfaces de guidage (15, 16) dont l'inclinaison correspond à l'angle d'inclinaison des deux demi-troncs de cône (3a, 3b) formant le double tronc de cône (3), et bordant partiellement ceux-ci, sur lesquelles les surfaces coniques des demi-troncs de cône (3a, 3b) viennent en appui coulissant.

2. Endoprothèse de l'articulation du genou selon la revendication 1, caractérisée en ce que les deux surfaces de glissement (4, 5) de l'implant tibial (2) sont séparées par deux nervures (6, 7) bordant le double tronc de cône (3) et s'étendant du côté dorsal vers le côté ventral, la nervure située du côté dorsal (6) présentant une largeur plus importante que la nervure située du côté ventral (7).

3. Endoprothèse de l'articulation du genou selon la revendication 1 ou 2, caractérisée en ce que les angles d'inclinaison des deux demi-troncs de cône (3a, 3b) sont sensiblement égaux.

4. Endoprothèse de l'articulation du genou selon l'une des revendications 1 à 3, caractérisée en ce que le double tronc de cône (3) est lié de façon amovible à l'implant tibial (2) grâce à une vis (8) le traversant axialement.

5. Endoprothèse de l'articulation du genou selon l'une des revendications 1 à 4, caractérisée en ce que le double tronc de cône (3) est constitué en un matériau synthétique résistant à l'abrasion.

6. Endoprothèse de l'articulation du genou selon l'une des revendications 1 à 5, caractérisée en ce que les surfaces de glissement (4, 5) de l'implant tibial (2) sont conformées en une garniture en matière synthétique (9), à faible coefficient de friction, liée de façon amovible à celui-ci.

7. Endoprothèse de l'articulation du genou selon l'une des revendications 1 à 6, caractérisée en ce que l'implant fémoral (1) est conformé en deux parties (1a, 1b), la première partie (1a) présentant une tige (10), au moyen de laquelle elle peut être ancrée et des surfaces d'appui (11) pour la deuxième partie (1b), qui peut être assemblée à la première partie (1a) au moyen d'un coinçage conique et qui porte les patins de glissement (12, 13).
